# EUROPEAN PATENT APPLICATION

(11) **EP 4 763 981 A1**
(43) Date of publication of application: **24.06.2026**
(21) Application number: 25225511.2
(22) Date of filing: 19.12.2025
(51) Int. Cl.: C12N 9/54

(54) **COMBINED DECOLORIZATION AND SULFATE AND PHOSPHATE REMOVAL FROM ENZYME CONTAINING FERMENTATION DERIVED SOLUTION**

(30) Priority: 20.12.2024 EP 24222395
(71) Applicant: BASF SE, 67056 Ludwigshafen am Rhein (DE)
(72) Inventor: Biselli, Andreas, 67056 Ludwigshafen am Rhein (DE); Kaeding, Thomas, 67056 Ludwigshafen am Rhein (DE); Wiese, Monika Theresia, 67056 Ludwigshafen am Rhein (DE)
(74) Representative: Altmann Stößel Dick Patentanwälte PartG mbB

(57) **Abstract**

The present invention relates to the field of purification of fermentation-derived products, in particular enzymes. The invention is directed to a method for purifying a fermentation-derived solution of an enzyme of interest comprising at least the following steps: a) providing a fermentation-derived solution of an enzyme of interest comprising 0.1 to 40 g/kg sulfate and 0 to 20 g/kg phosphate; b) contacting the enzyme solution with an anion exchange resin; c) obtaining a purified enzyme solution wherein the concentration of sulfate and/or phosphate is below the concentration of sulfate and/or phosphate in step a) and wherein the purified enzyme solution has a CIELAB L-value of above 60. Moreover, the invention contemplates a solution of an enzyme of interest obtained by or obtainable by said method.

## Description

The present invention relates to the field of purification of fermentation-derived products, in particular enzymes. The invention is directed to a method for purifying a fermentation-derived solution of an enzyme of interest comprising at least the following steps: a) providing a fermentation-derived solution of an enzyme of interest comprising 0.1 to 40 g/kg sulfate and 0 to 20 g/kg phosphate; b) contacting the enzyme solution with an anion exchange resin; c) obtaining a purified enzyme solution wherein the concentration of sulfate and/or phosphate is below the concentration of sulfate and/or phosphate in step a) and wherein the purified enzyme solution has a CIELAB L-value of above 60. Moreover, the invention contemplates a solution of an enzyme of interest obtained by or obtainable by said method.

A common way of producing a protein or enzyme of interest, particularly for industrial applications, is by fermentation in an appropriate host cell followed by purification from the fermentation media. The concentrated fermentation-derived protein solutions are usually obtained by means of several sequential process steps. Purified fermentation-derived protein solutions may be regarded as a purified raw material which is either used in liquid form or may occasionally be converted into a dry form and then put to appropriate uses.

Proteins of interest, in particular enzymes, in liquid form are increasingly used for liquid and gel-like detergents and cleaning compositions. Moreover, liquid forms of proteins of interest are highly demanded in the field of cosmetics, animal nutrition, the manufacture and processing of textiles and food production for converting raw materials to end products. High quality and highly pure protein fermentation products are desired for these applications. Undesired side components, in particular components that negatively impact product quality, product stability, product activity, product appearance and /or product odor are to be avoided in the protein fermentation product.

Moreover, high levels of sulfate and/or phosphate in formulations are undesirable, as they may lead to precipitation and have other undesired effects. Moreover, high levels of phosphate ions may often be undesired as they contravene environmental regulations in many countries.

DE3915277 A1 describes a process for cleaning and deodorizing aqueous enzyme solutions obtained by the fermentation of bacteria or fungi by the in situ production of a water-insoluble deposit. The adsorption of undesired admixtures to this deposit and subsequent solid/liquid separation is intended to improve the light color of the enzyme solution and the quantity of enzymes. This is achieved by adding to the enzyme solution an acid aqueous solution of an aluminum salt and, if desired, other precipitating agents and a separating off water-insoluble components, whereby a masking agent selected from the group: boric acids and/or their water-soluble salts; sulphurous acids and/or their water-soluble salts; hydroxycarboxylic acids and/or their water-soluble salts; multivalent alcohols may be added.

WO2004067557 A1 discloses a process comprising the steps of: providing a concentrated enzyme solution comprised of insoluble solids; separating the insoluble solids to produce a solids-free supernatant solution and contacting the supernatant solution with a strongly basic anion exchanger carried out with a bed volume of from 1 to 10 to produce a decolorized protein. The process requires concentrating the enzyme solution accompanied by removing insoluble solids. This may cause loss of enzyme product through precipitation.

Hence, there is a need to provide means and methods avoiding at least in part the drawbacks of the prior art as discussed above. In particular, there is still a need for improved purification processes that are simple and hence cost-effective and result in high levels of a purified enzyme of interest in a desired quality and having low levels sulfate and/or phosphate.

The technical problem underlying the present invention may be regarded as the provision of means and methods for complying with the aforementioned needs. The technical problem is solved by the embodiments characterized in the claims and herein below.

This problem is addressed by a method with the steps and features of the independent claims. Advantageous embodiments which might be realized in an isolated fashion or in any arbitrary combinations are listed in the dependent claims as well as throughout the specification.

Thus, the present invention relates to a method for purifying a fermentation-derived solution of an enzyme of interest. The method comprises the following steps which specifically may be performed in the given order or in a different order, such as in a reverse order. Thus, a different order may also be possible. Further, two or more process steps may be performed simultaneously or partially simultaneously. Further, one or more than one or even all of the method steps may be performed once or more than once or even repeatedly or continuously. The method may further comprise additional method steps which are not listed.

The method according to the invention comprises at least the following steps: a) providing a fermentation-derived solution of an enzyme of interest comprising 0.1 to 40 g/kg sulfate and 0 to 20 g/kg phosphate; b) contacting the enzyme solution with an anion exchange resin; c) obtaining a purified enzyme solution wherein the concentration of sulfate and/or phosphate is below the concentration of sulfate and/or phosphate in step a), preferably comprising less than 1 g/kg sulfate, less than 0.1 g/kg phosphate and wherein the purified enzyme solution has a CIELAB L-value of above 60.

The method according to the invention is advantageous as it provides a simple method for removing or at least decreasing undesired colored compounds and undesired sulfate and phosphate ions in a fermentation-derived solution of an enzyme of interest. In particular, the method allows for removing sulfate, phosphate and undesired colored compounds of the enzyme of interest in a single step. Surprisingly and advantageously, simultaneous sulfate and phosphate removal does not have a significant negative impact on the decolorization.

It is to be understood that as used in the specification and in the claims, "a" or "an" can mean one or more, depending upon the context in which it is used. Thus, for example, reference to "a host cell" can mean that at least one host cell can be utilized.

As used herein, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically are used only once when introducing the respective feature or element. In most cases, when referring to the respective feature or element, the expressions "at least one" or "one or more" are not repeated, non-withstanding the fact that the respective feature or element may be present once or more than once.

Further, as used herein, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The method according to the invention comprises a step a) of providing a fermentation derived solution of an enzyme of interest comprising 0.1 to 40 g/kg sulfate and 0 to 20 g/kg phosphate; preferably 0.1 g/kg to 10 g/kg sulfate and 0 to 10 g/kg phosphate.

The term "providing a fermentation-derived solution of an enzyme of interest" refers to any way of making available said fermentation-derived solution. The term specifically may refer, without limitation, to allocating said solution for processing in the method steps according to the invention. More specifically, said term refers to placing said solution into a suitable vessel or container to allow for performing the method steps foreseen by the invention. Said step of providing the fermentation-derived solution may be automated or may be performed manually. Means and methods for manual or automated provision of a fermentation-derived solution are known in the art. Typically, providing the fermentation-derived solution of an enzyme of interest is achieved continuously by pumping.

The term "enzyme of interest" as referred to herein refers to any enzyme, full length, or functional fragments thereof, which is intended to be produced in a fermentation process. The term "enzyme of interest", thus, encompasses enzymes, or functional fragments thereof as well as fusion proteins, labeled enzymes and the like. Functional fragments of an enzyme of interest refers to a fragment or part of said enzyme that is capable of providing, typically that provides, the enzymatic function of interest. Further details concerning the enzyme of interest are described elsewhere herein.

Preferably, the enzyme of interest is at least partially secreted by the host cells into the fermentation medium or is expressed intracellularly by the bacterial cells and may only unintentionally be released from the host cells into the fermentation medium, for example by lysis of the host cells. In other words, the enzyme of interest may be produced intracellularly.

A "host cell" as used herein is a cell that supports the production of the enzyme of interest, typically in a fermentation process. More typically, the host cell is recombinant, e.g. it preferably contains a heterologous polynucleotide also referred to as "expression construct" which is introduced by man by gene technology and with regard to a polynucleotide includes all those constructions brought about by man by gene technology / recombinant DNA techniques known in the art. In other words; according to the invention a host cell refer to a cell which serves as a host for an expression construct or vector for a gene encoding a enzyme of interest. Said expression construct may be a naturally occurring expression construct, a recombinantly introduced expression construct or a naturally occurring expression construct which has been genetically modified in the bacterial cell.

The term "recombinant" is known to the skilled artisan. In particular, "recombinant" (or transgenic) with regard to a cell or an organism means that the cell or organism contains a heterologous polynucleotide also referred to as "expression construct" which is introduced by man by gene technology and with regard to a polynucleotide includes all those constructions brought about by man by gene technology / recombinant DNA techniques known in the art.

The term "heterologous" (or exogenous or foreign or recombinant or non-native) polypeptide or protein is defined herein as a polypeptide that is not native to the host cell, a polypeptide or protein native to the host cell in which structural modifications, e.g., deletions, substitutions, and/or insertions, have been made by recombinant DNA techniques to alter the native polypeptide or protein, or a polypeptide/protein native to the host cell whose expression is quantitatively altered or whose expression is directed from a genomic location different from the native host cell as a result of manipulation of the DNA of the host cell by recombinant DNA techniques, or whose expression is quantitatively altered as a result of manipulation of the regulatory elements of the polynucleotide by recombinant DNA techniques e.g., a stronger promoter; or a polynucleotide native to the host cell, but integrated not within its natural genetic environment as a result of genetic manipulation by recombinant DNA techniques. The term "heterologously expressed" hence may typically refer to an enzyme of interest being expressed in a host cell from a heterologous expression construct. More typically, the host cell comprises the heterologous expression construct as a result of genetic manipulation by recombinant DNA techniques. These techniques are known in the art.

With respect to two or more polynucleotide sequences or two or more amino acid sequences, the term "heterologous" is used to characterize that the two or more polynucleotide sequences or two or more amino acid sequences are naturally not occurring in the specific combination with each other.

The term "nucleic acid" or "nucleic acid molecule" as used herein, includes reference to a deoxyribonucleotide (DNA) or ribonucleotide (RNA) polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids). A polynucleotide can be full-length or a sub-sequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are "polynucleotides" as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including among other things, simple and complex cells. Every nucleic acid sequence herein that encodes a polypeptide or protein such as the enzyme of interest as defined herein also, by reference to the genetic code, describes every possible silent variation of the nucleic acid. The term "conservatively modified variants" applies to both amino acid and nucleic acid sequences. With respect to particular nucleic acid sequences, the term "conservatively modified variants" refers to those nucleic acids which encode identical or conservatively modified variants of the amino acid sequences due to the degeneracy of the genetic code. The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation.

Typically, said host cell is selected from the group consisting of: a bacterial cell, a yeast cell, a fungal cell, an algal cell or a cyanobacterial cell, a non-human animal cell or a non-human mammalian cell, and a plant cell. More typically, the host cell can be selected from any one of the following organisms:

### Bacteria:

The bacterial host cell can, for example, be selected from the group consisting of the genera Escherichia, Klebsiella, Helicobacter, Bacillus, Lactobacillus, Streptococcus, Amycolatopsis, Rhodobacter, Pseudomonas, Paracoccus, Lactococcus, Ensifer or Pantoea.
gram positive: Bacillus, Streptomyces: Useful gram positive bacterial host cells include, but are not limited to, a Bacillus cell, e.g., Bacillus alkalophius, Bacillus amyloliquefaciens, Bacillus brevis, Bacillus circulans, Bacillus clausii, Bacillus coagulans, Bacillus firmus, Bacillus Jautus, Bacillus lentus, Bacillus licheniformis, Bacillus megaterium, Bacillus pumilus, Bacillus stearothermophilus, Bacillus subtilis, and Bacillus thuringiensis. Most preferred, the prokaryote is a Bacillus cell, preferably, a Bacillus cell of Bacillus subtilis, Bacillus pumilus, Bacillus licheniformis, or Bacillus lentus.

Some other preferred bacteria include strains of the order Actinomycetales, preferably, Streptomyces, preferably Streptomyces spheroides (ATTC 23965), Streptomyces thermoviolaceus (IFO 12382), Streptomyces lividans or Streptomyces murinus or Streptoverticillum verticillium ssp. verticillium. Other preferred bacteria include Rhodobacter sphaeroides, Rhodomonas pal-ustri, Streptococcus lactis. Further preferred bacteria include strains belonging to Myxococcus, e.g., M. virescens.
gram negative: Escherichia, Pseudomonas, Rhodobacter, Paracoccus, Ensifer or Pantoea species: Preferred gram negative bacteria are Escherichia coli, Pseudomonas sp., preferably, Pseudomonas purrocinia (ATCC 15958) or Pseudomonas fluorescens (NRRL B-11) or Pseudomonas denitrificans, Rhodobacter capsulatus or Rhodobacter sphaeroides, Paracoccus carotinifaciens, Paracoccus zeaxanthinifaciens, Pantoea ananatis, or Sinorhizobium meliloti also known as Ensifer meliloti.

Fungi:
Aspergillus, Fusarium, Trichoderma: The host cell may be a fungal cell. "Fungi" as used herein includes the phyla Ascomycota, Basidiomycota, Chytridiomycota, and Zygomycota as well as the Oomycota and Deuteromycotina and all mitosporic fungi. Representative groups of Ascomycota include, e.g., Neurospora, Eupenicillium (=Penicillium), Emericella (=Aspergillus), Eurotium (=Aspergillus), Thermothelomyces and the true yeasts listed below. Examples of Basidiomycota include mushrooms, rusts, and smuts. Representative groups of Chytridiomycota include, e.g., Allomyces, Blastocladiella, Coelomomyces, and aquatic fungi. Representative groups of Oomycota include, e.g. Saprolegniomycetous aquatic fungi (water molds) such as Achlya. Examples of mitosporic fungi include Aspergillus, Penicillium, Candida, and Alternaria. Representative groups of Zygomycota include, e.g., Rhizopus and Mucor.

Some preferred fungi include strains belonging to the subdivision Deuteromycotina, class Hyphomycetes, e.g., Fusarium, Humicola, Trichoderma, Myrothecium, Verticillum, Arthromyces, Caldariomyces, Ulocladium, Embellisia, Cladosporium or Dreschlera, in particular Fusarium oxysporum (DSM 2672), Humicola insolens, Trichoderma resii, Myrothecium verrucana (IFO 6113), Verticillum alboatrum, Verticillum dahlie, Arthromyces ramosus (FERM P-7754), Caldariomyces fumago, Ulocladium chartarum, Embellisia alli Dreschlera halodes or Thermothelomyces thermophiles (ATCC 42464) also referred to as Myceliophthora thermophila. Other preferred fungi include strains belonging to the subdivision Basidiomycotina, class Basidiomycetes, e.g. Coprinus, Phanerochaete, Coriolus or Trametes, in particular Coprinus cinereus f. micro-sporus (IFO 8371), Coprinus macrorhizus, Phanerochaete chrysosporium (e.g. NA-12) or Trametes (previously called Polyporus), e.g. T. versicolor (e.g. PR4 28-A).

Further preferred fungi include strains belonging to the subdivision Zygomycotina, class Mycoraceae, e.g. Rhizopus or Mucor, in particular Mucor hiemalis.

Yeast, Pichia, Saccharomyces: The fungal host cell may be a yeast cell. Yeast as used herein includes ascosporogenous yeast (Endomycetales), basidiosporogenous yeast, and yeast belonging to the Fungi Imperfecti (Blastomycetes). The ascosporogenous yeasts are divided into the families Spermophthoraceae and Saccharomycetaceae. The latter is comprised of four subfamilies, Schizosaccharomycoideae (e.g., genus Schizosaccharomyces), Nadsonioideae, Lipomycoideae, and Saccharomycoideae (e.g. genera Kluyveromyces, Pichia, and Saccharomyces). The basidiosporogenous yeasts include the genera Leucosporidim, Rhodosporidium, Sporidiobolus, Filobasidium, and Filobasidiella. Yeasts belonging to the Fungi Imperfecti are divided into two families, Sporobolomycetaceae (e.g., genera Sporobolomyces and Bullera) and Cryptococcaceae (e.g. genus Candida).

Eukaryotes:
Eukaryotic host cells further include, without limitation, a non-human animal cell, a non-human mammal cell, an avian cell, reptilian cell, insect cell or a plant cell.

Still more preferably, said host cell is a microbial cell, such as a fungal cell, an archaea cell, an algae cell, a protozoa cell or a bacterial cell; more preferably a fungal or a bacterial cell. Bacterial host cells are preferred.

Bacterial host cells may in principle be selected from the group of bacteria consisting of Bacillus, Streptomyces, Escherichia, Buttiauxella and Pseudomonas. In the method of the invention, the host cell is preferably a cell of a Bacillus species.

According to the method of the invention, preferably, the fermentation is a bacterial fermentation, more preferably the method comprises producing the enzyme of interest in a Bacillus host cell.

As mentioned herein, the host cell in the method according to the invention may be a Bacillus cell. The Bacillus cell may be a cell from any member of the bacterial genus Bacillus, preferably a host cell of Bacillus licheniformis, Bacillus subtilis, Bacillus alkalophilus, Bacillus amyloliquefaciens, Bacillus brevis, Bacillus circulans, Bacillus clausii, Bacillus coagulans, Bacillus firmus, Bacillus jautus, Bacillus lentus, Bacillus megaterium, Bacillus pumilus, Bacillus stearothermophilus, Bacillus thuringiensis or Bacillus velezensis. More preferably, the Bacillus host cell is a Bacillus licheniformis, Bacillus pumilus, or Bacillus subtilis cell, even more preferred Bacillus licheniformis or Bacillus subtilis cell, most preferably, Bacillus licheniformis host cell. Particular preferably, the Bacillus licheniformis is selected from the group consisting of Bacillus licheniformis as deposited under American Type Culture Collection number ATCC 14580, ATCC 31972, ATCC 53926, ATCC 53757, ATCC 55768, and under DSMZ number (German Collection of Microorganisms and Cell Cultures GmbH) DSM 13, DSM 394, DSM 641, DSM 1913, DSM 11259, and DSM 26543.

Typically, the host cell belongs to the species Bacillus licheniformis, such as a host cell of the Bacillus licheniformis strain as deposited under American Type Culture Collection number ATCC 14580 (which is the same as DSM 13, see Veith et al. "The complete genome sequence of Bacillus licheniformis DSM 13, an organism with great industrial potential." J. Mol. Microbiol. Biotechnol. (2004) 7:204-211). Alternatively, the host cell may be a host cell of Bacillus licheniformis strain ATCC 53926. Alternatively, the host cell may be a host cell of Bacillus licheniformis strain ATCC 31972. Alternatively, the host cell may be a host cell of Bacillus licheniformis strain ATCC 53757. Alternatively, the host cell may be a host cell of Bacillus licheniformis strain ATCC 53926. Alternatively, the host cell may be a host cell of Bacillus licheniformis strain ATCC 55768. Alternatively, the host cell may be a host cell of Bacillus licheniformis strain DSM 394. Alternatively, the host cell may be a host cell of Bacillus licheniformis strain DSM 641. Alternatively, the host cell may be a host cell of Bacillus licheniformis strain DSM 1913. Alternatively, the host cell may be a host cell of Bacillus licheniformis strain DSM 11259. Alternatively, the host cell may be a host cell of Bacillus licheniformis strain DSM 26543.

Fermentation processes for producing an enzyme of interest are known in the art and described for example in WO9102792 A1 and WO2021/001297 A1. Further details on the fermentation process are disclosed elsewhere herein.

The term "fermentation derived solution of an enzyme of interest" in line with the present invention is understood as any solution derivable from a fermentation process comprising the enzyme of interest. The enzyme of interest may be present in the fermentation derived solution in a solubilized state and/or in crystalline or precipitated or other solid form, typically the enzyme of interest is solubilized in the fermentation derived solution, i.e. in a solubilized state. Typically, said solution is an aqueous solution. More typically, a fermentation-derived solution of the enzyme of interest may be obtained by a solid liquid separation, for example filtration, crossflow filtration and/or centrifugation at the end of a fermentation process, for example after the time of harvest. The solid liquid separation may include a step of dilution, stabilization and/or other pretreatment. Even more typically, the fermentation-derived solution of the enzyme of interest is a permeate, a filtrate, a concentrate, a retentate, in particular an ultrafiltration retentate, or a supernatant, typically obtained after solid liquid separation.

Typically, the fermentation-derived solution is object to one or more concentration steps, such as ultrafiltration or evaporation or other means for concentration known in the art. More typically, the fermentation-derived solution of the enzyme of interest is obtained by one or more solid liquid separation steps known in the art as described elsewhere herein followed by one or more concentration steps mentioned above. More typically, the fermentation-derived solution of the enzyme of interest is obtained by one or more pre-treatment steps, such as dilution, pH adjustments, coagulation, flocculation, temperature adjustments, addition of stabilizing chemicals, or combinations of aforementioned, followed by one or more solid-liquid separation steps, followed by one or more concentration steps, known in the art. As noted elsewhere herein, said steps may specifically be performed in the given order or in a different order, such as in a reverse order. Thus, a different order may also be possible. Further, two or more process steps may be performed simultaneously or partially simultaneously. Further, one or more than one or even all of the method steps may be performed once or more than once or even repeatedly or continuously. The method may further comprise additional method steps which are not listed.

In a typical embodiment, the method comprises obtaining the fermentation derived solution by at least the following steps: (i) pretreatment such as coagulation, precipitation, adjustment of the pH, adjustment of the temperature and/or dilution; (ii) solid-liquid separation, typically following the pretreatment; and (iii) concentration of the resulting solution.

In the alternative, the fermentation broth at the time of harvest may be directly subjected as the fermentation-derived solution to the method according to the invention.

The fermentation derived solution of an enzyme of interest may specifically have a CIELAB L-value of about 60 or even below, more specifically a CIELAB L-value between 60 and 80. It is to be understood that, more specifically, the fermentation derived solution of an enzyme of interest has an L-value that is significantly below the CIELAB L-value of the purified enzyme solution. The CIELAB L-value of the fermentation derived solution is preferably at least 5 units lower than the L-value of the purified enzyme solution in step c), more preferably at least 7, still more preferably at least 10 units, at least 15 units, at least 20 unit, or at least 25 units lower than the L-value of the purified enzyme solution obtained in step c).

The fermentation-derived solution of an enzyme of interest comprises 0.1 to 40 g/kg sulfate and 0 to 20 g/kg phosphate. Moreover, the fermentation-derived solution of an enzyme of interest may comprise undesired colored compounds which typically account for an undesired coloration of the said solution. The amount of sulfate in the fermentation-derived solution may be determined by any means and methods known in the art such as spectrophotometric measurements, such as UV-Vis spectrophotometry, elemental analysis, ion chromatography, spectroscopic determination.

The present invention is in particular advantageous as it is suitable for removing undesired residual sulfate ions and, if present, phosphate ions together and for decolorizing the solution in a single step. Surprisingly and advantageously, sulfate and/or phosphate may be removed together with the undesired colored compounds without a significant negative impact on the decolorization.

Typically, the fermentation-derived solution of an enzyme of interest comprises 0.1 to 10 g/kg sulfate; more typically 0.05 to 5 g/kg sulfate. The fermentation-derived solution of an enzyme of interest may comprise 0.05 to 10 g/kg phosphate; 0 to 3 g/kg phosphate or may comprise 0 to 1 g/kg phosphate.

These levels of sulfate and/or phosphate ions in the fermentation-derived solution are typically undesired as they may interfere with downstream purification and formulation steps and/or environmental regulations. Hence, the method according to the invention is advantageous as it provides a simple way of reducing said levels to acceptable limits and thereby further decreases coloration of the fermentation-derived solution of the enzyme of interest. The acceptable limits of sulfate and phosphate in the purified solution are described elsewhere herein in more details.

The amount of phosphate may be determined by means and methods as known in the art. For example by spectrophotometry, high performance liquid chromatography, spectroscopic determination, colorimetric detection and commercially available test kits for example by Bayrol Deutschland GmbH (Germany).

According to the present invention, preferably, the enzyme of interest is produced by a host cell, more preferably said host cell comprising an expression construct carrying a nucleic acid sequence encoding for the enzyme of interest, during the fermentation process.

A "fermentation process" comprises the cultivation of host cells, in particular the bacterial cells, more particularly the Bacillus cells, in a suitable fermentation medium, also referred to as "cultivation medium". "Cultivation of the cells" or "growth of the cells" is not understood to be limited to an exponential growth phase but can also include the physiological state of the cells at the beginning of growth after inoculation and during a stationary phase. Typically the cultivation may take place at a cultivation temperature suitable for supporting growth of the cells. More typically, the cultivation temperature may lay within the range of 25 °C to 45 °C. The fermentation process may usually take place in a fermenter, preferably in a fermenter with a volume scale which is at least 1 m³.

In line with the present invention, the method for purifying may be performed at the end of a fermentation process, in particular after harvest, such as when a desirable amount of enzyme of interest is reached. Alternatively, the method for purifying may be performed in line, i.e. simultaneously with the fermentation process, for example by employing partial harvesting.

An industrially relevant fermentation process encompasses a fermentation process on a volume scale which is 1-500 m³ with regard to the nominal fermenter size, preferably 5-500 m³, more preferably 10-500 m³, even more preferably 25-500 m³, most preferably 50-500 m³. In other words, an industrially relevant fermentation process encompasses a fermentation process on a volume scale which is at least 1000 L with regard to the nominal fermenter size, preferably at least 5,000 L, more preferably at least 10,000 L, even more preferably at least 25,000 L, still even more preferably at least 50,000 L and most preferably 50,000-500,000 L.

Preferably, the enzyme of interest is classified as an oxidoreductase (EC 1), a transferase (EC 2), a hydrolase (EC 3), a lyase (EC 4), an isomerase (EC 5), or a ligase (EC 6) (EC-numbering according to Enzyme Nomenclature, Recommendations (1992) of the Nomenclature Committee of the International Union of Biochemistry and Molecular Biology including its supplements published 1993-1999). In a preferred embodiment, the enzyme of interest is an enzyme suitable to be used in detergents.

Most preferably, the enzyme is a hydrolase (EC 3), preferably, a glycosidase (EC 3.2) or a peptidase (EC 3.4). Especially, preferred enzymes are enzymes selected from the group consisting of an amylase (in particular an alpha-amylase (EC 3.2.1.1)), a cellulase (EC 3.2.1.4), a lactase (EC 3.2.1.108), a mannanase (EC 3.2.1.25), a lipase (EC 3.1.1.3), a phytase (EC 3.1.3.8), a nuclease (EC 3.1.11 to EC 3.1.31), and a protease (EC 3.4); in particular amylase, alpha-amylase, glucoamylase, pullulanase, protease, metalloprotease, peptidase, lipase, cutinase, acyl transferase, cellulase, endoglucanase, glucosidase, cellubiohydrolase, xylanase, xyloglucantransferase, xylosidase, mannanase, phytase, phosphatase, xylose isomerase, glucoase isomerase, lactase, acetolactate decarboxylase, pectinase, pectin methylesterase, polygalacturonidase, lyase, pectate lyase, arabinase, arabinofuranosidase, galactanase, a laccase, peroxidase and an asparaginase.

Enzymes of particular interest may further be selected from: protease, oxidoreductase, transferase, hydrolase, lyase, isomerase, ligase, aminopeptidase, amylase, asparaginase, carbohydrase, carboxypeptidase, catalase, cellulase, chitinase, cutinase, cyclodextrin glycosyltransferase, deoxyribonuclease, esterase, alpha-galactosidase, betagalactosidase, glucoamylase, alpha-glucosidase, beta-glucosidase, hyaluronic acid synthase, invertase, laccase, lipase, mannanase, mannosidase, mutanase, oxidase, a pectinolytic enzyme, peroxidase, phytase, polyphenoloxidase, ribonuclease, transglutaminase, dispersin, and xylanase.

More particularly, the enzyme may be selected from the group consisting of: amylase, protease, lipase, lactase, mannanase, phytase, xylanase, phosphatase, glucoamylase, nuclease, and cellulase, even more particularly selected from the group consisting of: amylase, protease, lipase, mannanase, phytase, xylanase, phosphatase, glucoamylase, nuclease, and cellulase, more preferably the enzyme of interest is selected from amylase, and protease; even more preferably the enzyme of interest is a protease, for example a serine protease such as subtilisin protease; even more preferably the enzyme of interest is an amylase, such as alpha-amylase.

In line with the present invention, the enzyme of interest has an isoelectric point in the range of 4 to 10; preferably in the range of 6 to 10 if the enzyme of interest is a subtilisin protease, in the range of 4 to 7.5 if the enzyme is an amylase; typically said isoelectric points relating to the mature enzyme.

Further, the method according to the invention comprises a step b) of contacting the enzyme solution of the enzyme of interest with an anion exchange resin.

The step of contacting the enzyme solution of the enzyme of interest with an anion exchange resin may refer to any way of applying the enzyme solution to the anion exchange resin; typically in a continuous or discontinuous way, such as pumping; pouring, dripping, transferring via microfluidics, and the like. Alternatively, the resin may be added to the fermentation derived solution of an enzyme of interest in a suitable amount.

Suitable anion exchange resins are known in the art. It is understood that an anion exchange resin in line with the present invention specifically refers to a resin that is capable of exchanging anions, in particular by interacting with functional groups of the resin. Typically, the resin is strongly basic; more typically, it is a polymer comprising functional groups, e.g. groups capable of exchanging anions. Suitable polymers may include polystyrene, sulfonate and polyacrylate. The anion exchange resin may generally not be limited to a certain type of functional groups. Said functional groups may specifically carry a positive charge over a wide pH range. The anion exchange resin typically comprises a strongly basic resin comprising ammonium, in particular quaternary ammonium groups, or substituted ammonium groups. More typically, said functional groups are loaded with counter-ions selected from chloride, hydroxide, formiate, acetate, carbonate and/or similar anions which are even more typically being exchanged in the anion exchange process. A person of skill in the art knows how to select a suitable counter-ion depending on the separation to be achieved. Moreover, the resin may comprise a porous polymer matrix. Said matrix may generally not be limited to a certain type of polymer or functional groups. However, a suitable polymer matrix may be, in particular a styrene matrix or an acrylic matrix, or the like; more particularly, a matrix comprising or consisting of a styrene-di-vinylbenzene copolymer. The polymer matrix may be porous, preferably macroporous. Still more typically, the anion exchange resin may be part of an anion exchange chromatography column or of an adsorption column; still more typically of an anion exchange chromatography column.

More typically, the anion exchange is performed on an anion exchange resin comprising quaternary ammonium groups. Still more typically, the resin comprises particles of a mean particle size in the range of 0.1 to 5 mm, even more typically in the range of 0.3 to 3 mm, specifically according to the manufacturer's determination. The mean particle size can be determined as known in the art, specifically as specified by the manufacturer. Typically, the mean particle size can be determined using dynamic light scattering, for example using a Malvern Mastersizer 3000. The particle size may typically have an influence on the pressure in an anion exchange column and hence may influence the applied pressure and/or dimensions of the column. Suitable anion exchange resins are commercially available and include for example DuPont^{™} AmberLite^{™} SCAV3 Cl.

The step of contacting the solution of the enzyme of interest with an anion exchange resin may typically include performing an anion exchange with said solution. In other words, in step b) an anion exchange is performed with said enzyme solution. More typically, the resin exchanges anions, in particular undesired anions, present in the fermentation derived solution; with anions bound to the anion exchange resin. Even more typically, undesired anions and potentially further undesired compounds are adsorbed to the resin and may be released during regeneration of the resin.

Further, said step may typically remove or at least reduce undesired colored compound. In other words, it may enhance clarity of the solution. Indeed, said anion exchange is considered advantageous as in a single step, undesired sulfate, undesired phosphate as well as undesired colored compounds may be removed. Without wishing to be bound by theory it is thought by the inventors that said undesired compounds adsorb to the anion exchange resin. The molecular interactions between undesired colored compounds and the anion exchange resin may be of any type, including hydrophobic and/or ionic interactions.

Said anion exchange in step b) may specifically be performed on an anion exchange chromatography column or on an adsorption column, more specifically on an anion exchange chromatography column ; even more specifically it may be performed using a bed volume between 0.5 m³ and 20 m³, preferably between 0.8 m³ and 15 m³, more preferably between 1 m³ and 8 m³. More preferably said bed volume may be distributed in one anion exchange column or one adsorption column or between several anion exchange columns or adsorption columns for example between a first and a second anion exchange column. The mode of running the anion exchange may be conventional, e.g. loading of a column, followed by washing and regenerating; or may be any other mode, e.g. loading of a first and a second column in parallel or in series. A different mode of running the column may comprise loading the enzyme solution to a first column up to its loading limit, the first column being in series with a second column. Any residual enzyme solution may then be directly loaded to the second column while the first column may be washed and regenerated. The loading limit or load capacity may typically refer to the loading volume of enzyme solution at which the anion exchange resin in the column reaches saturation. Said mode of running is advantageous as the load capacity of the first column may be completely exploited; further, the first column may be regenerated even before the run is completed.

Moreover, the method comprises a step c) of obtaining a purified solution of the enzyme of interest, preferably comprising less than 1 g/kg of sulfate, less than 0.1 g/kg phosphate, more preferably comprising less than 0.1 g/kg of sulfate, less than 0.05 g/kg phosphate; and wherein the purified solution has a CIELAB L-value of above 60; preferably above 70, more preferably above 80, even more preferably above 90. It is to be understood that the concentration of sulfate and/or phosphate in the purified solution is below the concentration in step a). High CIELAB L values reflect an increasingly light color of the solution. Said CIELAB L-values above 60 or higher are hence advantageous as the measured product has an appearance being sufficiently light.

CIELAB values can be determined by any means known in the art. Typically, for determining CIELAB values, a spectrophotometer is used, such as a Hach Lange DR 6000 or other commercially available spectrophotometer; more typically, the CIELAB value is determined spectrophotometrically, i.e. in a spectrophotometer, by measuring a sample of the purified solution, in a photometric cuvette such as a 1 mL polystyrene cuvette. The purified solution may preferably filtered using a 0.2 µm syringe filter. More preferably, by using a 0.2 µm syringe filter, the purified solution may be turned into an optically clear solution, even more preferably with an NTU below 20.

The CIELAB scale refer to a uniform color scale that is commonly used today (CIE International Commission on Illumination, Recommendations on Uniform Color Spaces, Color-Difference Equations, Psychometric Color Terms, Supplement No. 2 to CIE Publication No. 15, Colorimetry, 1971 and 1978).

The CIELAB L-value of the purified enzyme solution in step c) is preferably at least 5 units higher than the L-value of the fermentation derived solution of the enzyme in step a), more preferably at least 7, still more preferably at least 10 units, at least 15 units, at least 20 unit, or at least 25 units higher than the L-value of the fermentation derived solution of the enzyme in step a).

In line with the present invention, the CIELAB a- and/or the b-value of the purified enzyme solution are typically lower than the CIELAB a- and/or the b-value of the fermentation-derived solution of an enzyme of interest.

More typically, the CIELAB a-value of the purified enzyme solution in step c) is at least 1 unit lower than the a-value of the fermentation derived solution of the enzyme of interest in step a), preferably at least 2, more preferably at least 3, at least 4 units, at least 5 units, at least 6 units, at least 7 units, at least 8 units, at least 9 units, at least 10 units, at least 11 units, at least 12 units, at least 13 units, at least 14 units, at least 15 units, at least 16 units, at least 17 units, at least 18 units, at least 19 units, or at least 20 units lower than the a-value of the fermentation derived solution of the enzyme of interest in step a).

Even more typically, the CIELAB b-value of the purified enzyme solution in step c) is at least 5 units lower than the b-value of the fermentation derived solution of the enzyme of interest in step a), preferably at least 10, more preferably at least 15 units, at least 20 units, at least 25 units, at least 30 units, at least 35 units, or at least 40 units lower than the b-value of the fermentation derived solution of the enzyme of interest in step a).

The final CIELAB values for a and/or b in the purified solution are preferably in the range of 5 to -10 for a, more preferably 2 to - 5 for a, even more preferably in the range of 1 to -3 for a; and/or in the range of 50 to 5 for b; and/or more preferably 40 to 5 for b, even more preferably 30 to 5 for b.

Typically, the enzyme, in particular the protease, in step c) has an activity of at least 95% of the enzyme or protease in the fermentation-derived solution in step a).

Means and method for determining enzyme activity are known in the art. In line with the invention, protease activity may be determined using a colorimetric assay.

Typically, the purified enzyme solution comprises less than 0.05 g/kg phosphate, more typically less than 0.02 g/kg phosphate. Even more typically, the purified solution of the enzyme of interest comprises less than 1 g/kg sulfate and /or less than 0.1 g/kg phosphate; more preferably less than 0.5 g/kg sulfate and /or less than 0.1 g/kg phosphate more preferably less than 0.1 g/kg sulfate and /or less than 0.05 g/kg phosphate.

In line with the invention, the method typically does not require the addition of an aluminum sulfate; hence, the fermentation-derived solution of an enzyme of interest sulfate does in particular not comprise aluminum sulfate. The method according to the invention preferably does not comprise a step for removing of particulates or solid material. Typically, the fermentation-derived solution is free of particulates and solid material and hence a step for removing the same is not required.

In an embodiment, the method according to the invention consists of steps a) to c) as described elsewhere herein.

The method according to the invention may comprise one or more of the following additional steps: of ultra-centrifugation, evaporation, ultrafiltration, centrifugation, spray draying, granulation, freeze-drying, stabilization, crystallization, precipitation, adsorption, chromatographic purification such as affinity chromatography, and the like.

The method according to the invention may further comprise a step of adding an enzyme stabilizing system to the purified enzyme solution. Preferably, the enzyme stabilizing system comprises at least one compound selected from the group consisting of polyols (preferably, 1,3-propanediol, ethylene glycol, glycerol, 1,2-propanediol, or sorbitol), inorganic salts (preferably, CaCl₂, MgCl₂, or NaCl), short chain (preferably, C1-C3) carboxylic acids or salts thereof (preferably, formic acid, formateacetic acid, acetate, or lactate), borate, boric acid, boronic acids (preferably, 4-formyl phenylboronic acid (4-FPBA)), peptide aldehydes (preferably, Benzyloxycarbony-VAL-H (Z-VAL-H or Cbz-VAL-H) or Benzyloxycarbony-GAY-H (Z-GAY-H or Cbz-GAY-H), peptide acetals, and peptide aldehyde hydrosulfite adducts. Preferably, the enzyme stabilizing system comprises a combination of at least two of the compounds selected from the group consisting of salts, polyols, and short chain carboxylic acids and preferably one or more of the compounds selected from the group consisting of borate, boric acid, boronic acids (preferably, 4-formyl phenylboronic acid (4-FPBA)), peptide aldehydes, peptide acetals, and peptide aldehyde hydrosulfite adducts. In a particularly preferred embodiment, the stabilizing system comprises a protease inhibitor, preferably selected from borate, boric acid, boronic acids (preferably, 4-FPBA), peptide aldehydes (preferably, peptide aldehydes like Z-VAL-H or Z-GAY-H), peptide acetals, and peptide aldehyde hydrosulfite adducts, preferably the protease inhibitor is a peptide aldehyde, preferably Z-VAL-H or Z-GAY-H. In one embodiment, the stabilizing system does not comprise a protease inhibitor. Preferably, the solution is boron-free. Preferably, the purified enzyme solution comprises a calcium salt, preferably calcium chloride.

In one embodiment, the purified enzyme solution comprises a protease inhibitor, preferably a peptide aldehyde, in amounts in the range of about 0.05% to 0.8% by weight relative to the total weight of the purified enzyme solution. Preferably, the peptide aldehyde is comprised in amounts in the range of about 0.1% to 0.6% by weight, of about 0.1% to 0.5% by weight, of about 0.1% to 0.4%, or of about 0.1% to 0.35% by weight, all relative to the total weight of the purified solution.

In the method according to the invention, the enzyme of interest may be in an inactivated state throughout the purification process; for example during steps a) to c). The term "inactivated state" may refer to any state of reduced enzymatic activity. Said state may be achieved by adapting the pH or by other suitable adaptions such as temperature reduction.

A typical range of the temperature throughout the purification process is between 5 and 60 °C, more typically between 5 and 50 °C, even more typically between 5 and 40 °C, still even more typically between 5 and 35 °C.

Preferably, the purified enzyme solution comprises a protease in a concentration of 2 to 400 g/kg, more preferably 30 to 150 g/kg; more preferably 80 to 120 g/kg of the purified solution.

The method according to the invention may comprise a step of adjusting the pH in the fermentation-derived solution of an enzyme of interest and/or in the purified enzyme solution.

Adjusting the pH may be done by stepwise addition of an acid to lower the pH value or of a base to increase the pH value until the desired pH value is reached. As a suitable acid hydrochloric acid, sulfuric acid, acetic acid or formic acid may be used, typically hydrochloric acid, acetic acid or formic acid; a suitable base may be sodium hydroxide, potassium hydroxide or ammonium hydroxide. The addition may be automated or performed manually.

More particularly, the pH of the fermentation derived solution of the enzyme of interest is at least 0.5 pH units below the enzyme's isoelectric point. Isoelectric points of typical enzymes of interest are specified elsewhere herein.

The invention further contemplates a solution of an enzyme of interest obtained by or obtainable by the method according to the invention. Said enzyme solution is advantageous as it has desirably low amounts of sulfate and phosphate and is light or very light in color.

The explanations and interpretations of the terms given above in this specification apply for all embodiments characterized herein. Summarizing and without excluding further possible embodiments, the following embodiments are particular preferred embodiments according to the present invention.

### EMBODIMENTS:

Embodiment 1: A method for purifying a fermentation derived solution of an enzyme of interest comprising at least the following steps:
a) providing a fermentation derived solution of an enzyme of interest comprising 0.1 to 40 g/kg sulfate, and 0 to 20 g/kg phosphate
b) contacting the enzyme solution of the enzyme of interest with an anion exchange resin;
c) obtaining a purified solution of the enzyme of interest; wherein the concentration of sulfate and/or phosphate is below the concentration of sulfate and/or phosphate in step a); typically comprising less than 0.1 g/kg sulfate, and wherein the purified enzyme solution has a CIELAB L-value of above 60.

Embodiment 2: The method according to embodiment 1, wherein the purified enzyme solution comprises less than 0.05 g/kg phosphate.

Embodiment 3: The method according to embodiment 1 or 2, wherein the enzyme of interest is a protease.

Embodiment 4: The method according to embodiment 3, wherein the protease is a serine protease, more preferably subtilisin protease.

Embodiment 5: The method according to any of embodiments 2 to 4, wherein the protease in step c) has an activity of at least 95% of the protease in the fermentation-derived solution in step a).

Embodiment 6: The method according to any of embodiments 1 to 4, wherein the enzyme of interest has an isoelectric point in the range of 5 to 10; preferably in the range of 6 to 10 if the enzyme of interest is a protease, in the range of 4 to 7.5 if the enzyme is an amylase.

Embodiment 7: The method according to any of embodiments 1 to 6, wherein the purified solution of the enzyme of interest comprises less than 1 g/kg sulfate and /or less than 0.1 g/kg phosphate.

Embodiment 8: The method according to any of the embodiments 1 to 7, wherein the method does not comprise a step for removing particulates or solid material.

Embodiment 9: The method according to any of the embodiments 1 to 8, further comprising a step of adding an enzyme stabilizing system to the purified enzyme solution.

Embodiment 10: The method according to embodiment 9, wherein the stabilizing system comprises at least one compound selected from the group consisting of polyols (preferably, 1,3-propanediol, ethylene glycol, glycerol, 1,2-propanediol, or sorbitol), inorganic salts (preferably, CaCl2, MgCl2, or NaCl), short chain (preferably, C1-C3) carboxylic acids or salts thereof (preferably, formic acid, formate, acetic acid, acetate, or lactate), borate, boric acid, boronic acids (preferably, 4-formyl phenylboronic acid (4-FPBA)), peptide aldehydes (preferably, Benzyloxycarbony-VAL-H (Z-VAL-H or Cbz-VAL-H) or Benzyloxycarbony-GAY-H (Z-GAY-H or Cbz-GAY-H), peptide acetals, and peptide aldehyde hydrosulfite adducts.

Embodiment 11: The method according to any of the preceding embodiments, wherein the L-value of the purified enzyme solution in step c) is at least 5 units higher than the L-value of the fermentation derived solution of the enzyme in step a), preferably at least 7, more preferably at least 10 units higher than the L-value of the fermentation derived solution of the enzyme in step a).

Embodiment 12: The method according to any of the preceding embodiments, wherein the enzyme of interest is in an inactivated state throughout the purification process.

Embodiment 13: The method according to any of the preceding embodiments, wherein the method further comprises one or more of the following additional steps: ultracentrifugation, evaporation, ultrafiltration, centrifugation, spray draying, granulation, freeze-drying, stabilization, crystallization, precipitation, adsorption, chromatographic purification such as affinity chromatography, and the like.

Embodiment 14: The method according to any of the preceding embodiments, wherein the anion exchange is performed on an anion exchange resin, preferably on an anion exchange column, comprising ammonium groups or substituted ammonium groups; more preferably comprising quaternary ammonium groups, even more preferably on an anion exchange column.

Embodiment 15: The method according to any of the preceding embodiments, wherein the anion exchange is performed on an anion exchange column or on an adsorption column comprising an anion exchange resin having an acrylic or styrenic matrix.

Embodiment 16: The method according to the preceding embodiment, wherein the anion exchange in step c) is preformed using a bed volume between 0.5 m³ and 20 m³, preferably between 1 m³ and 8 m³.

Embodiment 17: The method according to any of the preceding embodiments, wherein the temperature is between 5 and 60 °C throughout the purification process.

Embodiment 18: The method according to any of embodiments 2 to 15, wherein the purified enzyme solution comprises a protease in a concentration of 2 to 400 g/kg, preferably 30 to 150 g/kg.

Embodiment 19: The method according to any of the preceding embodiments, further comprising a step of adjusting the pH in the purified enzyme solution.

Embodiment 20: The method according to any of the preceding embodiments, wherein the pH of the fermentation derived solution of the enzyme of interest is at least 0.5 pH units below the enzyme's isoelectric point.

Embodiment 21: The method according to any of the preceding embodiments, wherein the CIELAB a- and/or the b-value of the purified enzyme solution is lower than the CIELAB a- and/or the b-value of the fermentation-derived solution of an enzyme of interest.

Embodiment 22: The method according to any of the preceding embodiments, wherein the CIELAB a-value of the purified enzyme solution in step c) is at least 1 unit lower than the a-value of the fermentation derived solution of the enzyme of interest in step a), preferably at least 2 units, more preferably at least 3 units lower than the a-value of the fermentation derived solution of the enzyme of interest in step a).

Embodiment 23: The method according to any of the preceding embodiments, wherein the CIELAB b-value of the purified enzyme solution in step c) is at least 5 units lower than the b-value of the fermentation derived solution of the enzyme of interest in step a), preferably at least 10 units, more preferably at least 15 units lower than the b-value of the fermentation derived solution of the enzyme of interest in step a).

Embodiment 24: The method according to any of the preceding embodiments, wherein the method consists of steps a) to c).

Embodiment 25: The method according to any of the preceding embodiments, wherein the fermentation derived solution comprises undesired colored compounds.

Embodiment 26: A solution of an enzyme of interest obtained by or obtainable by the method according to any of the preceding embodiments.

The invention is not limited to one of the embodiments described above, but is modifiable in a great variety of ways. Those skilled in the art recognize that the embodiments according to the invention can easily be adapted without departing from the scope of the invention. Further characteristics, details and advantages of the invention follow from the wording of the claims, from the following description of practical examples and from the figures.

The content of all literature references cited in this patent application is hereby included by reference to the respective specific disclosure content and in its entirety.

The following examples serve to illustrate the invention. They must not be interpreted as limiting with regard to the scope of protection.

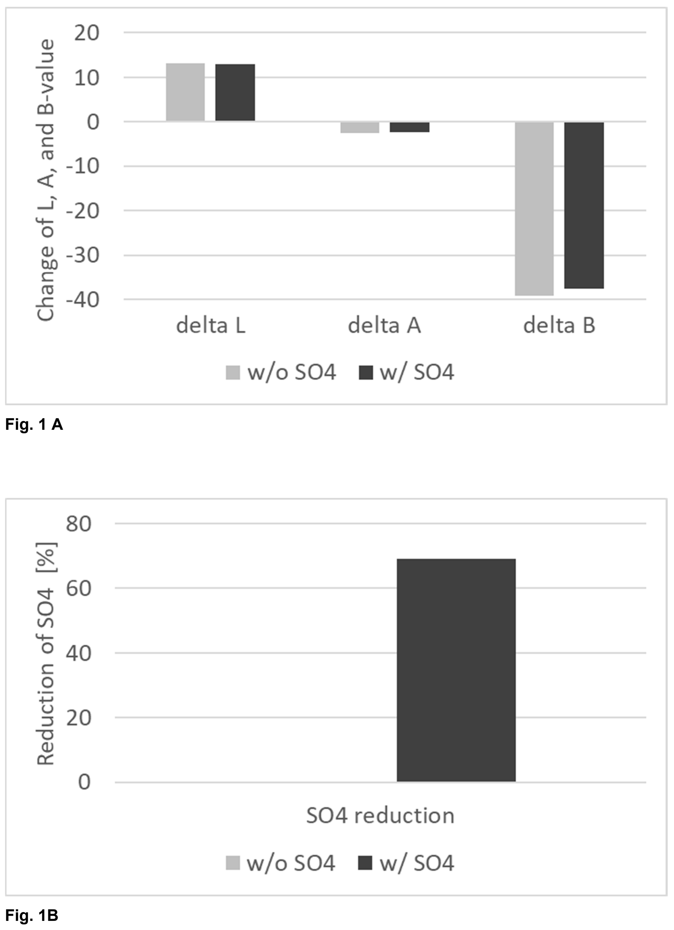

**Figure 1.** Figure 1 A depicts the change of the CIELAB color values L, A and B of samples of a fermentation derived solution without sulfate (w/o SO4) and with spiked sulfate (w/ SO4). In both samples, a strong base anion exchange resin was added. No significant influence of the sulfate concentration on color removal was observed. Figure 1 B depicts the reduction of sulfate in respective samples shown in Figure 1 A. The figure demonstrates that 69% of spiked sulfate was removed from the sample without influencing the color removal performance (cf. Figure 1 A).

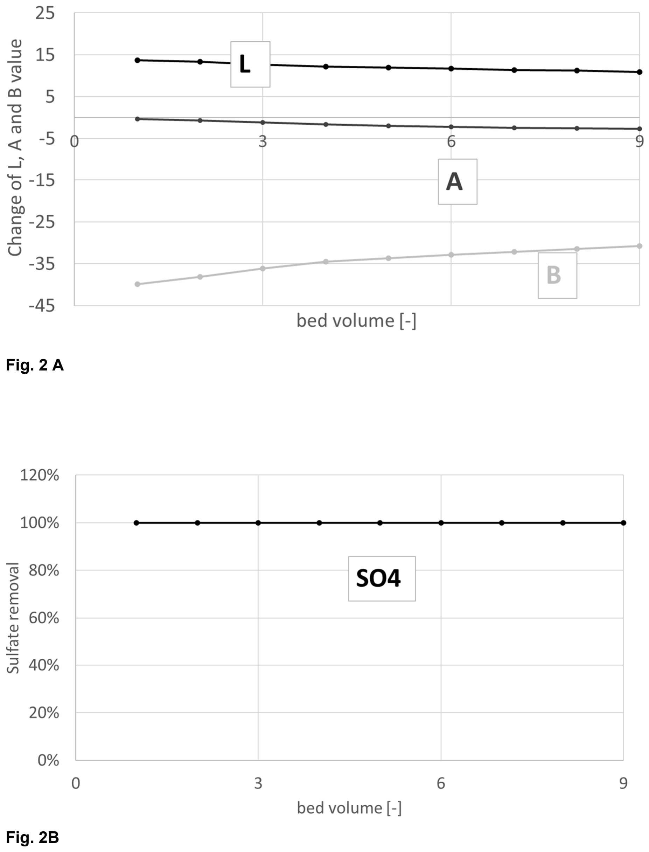

**Figure 2.** Figure 2 depicts the results of a breakthrough experiment. In Figure 2 A, the change of the CIELAB color values L, A and B of a fermentation derived solution pumped through an adsorption column filled with a strong base anion exchange resin is shown. In Figure 2 B, the reduction of the sulfate concentration of respective solution is shown. The figure demonstrates the removal of both, sulfate and color components.

### EXAMPLE 1

A fermentation was performed as described in EP3927837A1. 20 g of a fermentation derived solution without (w/o) sulfate (< 0,05 g/kg) purified from the fermentation broth via pre-treatment, solid-liquid separation and concentration steps, known in the art, was subjected to 2.5 g of the strong base anion exchange resin DuPont^{™} AmberLite^{™} SCAV3 in a stirred beaker for 24h. The pH value of the fermentation derived solution was approx. 1.5 - 1.6 pH units below the enzymes isoelectric point. CIELAB color values of the fermentation derived solution were measured before and after incubation using a Lico 690 Spectral colorimeter. Measured samples were previously sterile filtered using 0.2 µm syringe filters ensuring optically clear solutions.

The experiment was repeated with the same fermentation derived solution, which was spiked with 5 g/kg sulfate (w/) before adding the strong base anion exchange resin. Again, CIELAB color values L, A and B were measured before and after incubation as described above. Further, the sulfate concentration before and after incubation was measured spectroscopically.

Results are depicted in Figure 1A and 1B. Additionally, Table 1 shows the reduction of color of the experiment without spiked SO4 in the fermentation derived solution as well as the reduction of color and SO4 of the experiment with 5 g/kg SO4 spiked into the fermentation derived solution.

Delta values indicate the increase (positive values) or decrease (negative values) in respective values and/or concentrations due to the incubation with respective amounts of the strong base anion exchange resin.

| Ferm. Derived solution | Amount of resin added | Delta L | Delta A | Delta B | Delta SO₄ |
|---|---|---|---|---|---|
| [g] | [g] | | | | [g/kg] |
| 20 | 2.5 | 13.1 | -2.5 | -39.2 | w/o spiked SO4 |
| 20 | 2.5 | 12.9 | -2.4 | -37.6 | -3.2 |

The results clearly indicate that both, colored compounds and sulfate, adsorb to the strong base anion exchange resin. To our surprise, the adsorption of sulfate did not influence the removal of colored compounds, which is indicated by almost identical changes of the L, A and B values in both experiments.

### EXAMPLE 2

A fermentation derived solution purified from a fermentation broth via pre-treatment, solid-liquid separation and concentration steps, known in the art, was spiked with 1 g/kg sulfate. The fermentation was performed as described in EP3927837A1. The pH value of the fermentation derived solution was approx. 1.7 pH units below the enzymes isoelectric point. CIELAB color values of the fermentation derived solution were measured using a Lico 690 Spectral colorimeter. Measured samples were sterile filtered using 0.2 µm syringe filters ensuring optically clear solutions.

The SO4 spiked fermentation derived solution was continuously pumped through an adsorption column filled with the strong base anion exchange resin DuPont^{™} AmberLite^{™} SCAV3. Samples at the column outlet were taken for 9 bed volumes after each bed volume and analyzed for coloration by CIELAB value measurements and for sulfate, as described in example 1. Figure 2A shows the change of the color values L, A and B measured at the column outlet compared to the inlet values of the fermentation derived solution, over the fed bed volumes. Further, Figure 2B shows the reduction of the sulfate concentration at the column outlet. Table 3 shows the respective values for the reduction of color and SO4 at the column outlet compared to respective values of the fermentation derived solution. Delta values indicate the increase (positive values) or decrease (negative values) in respective values at the column outlet compared to the fermentation derived solution pumped into the adsorption column.

The results clearly indicate a full adsorption of sulfate, while removing significant parts of colored compounds measured via changes in L, A and B values. Values for phosphate were below the detection limit.

| Bed volume | Delta L | Delta A | Delta B | Sulfate removal |
|---|---|---|---|---|
| | | | | [%] |
| 1 | 13.7 | -0.3 | -39.9 | 100 |
| 2 | 13.4 | -0.7 | -38.1 | 100 |
| 3 | 12.7 | -1.2 | -36.1 | 100 |
| 4 | 12.2 | -1.6 | -34.5 | 100 |
| 5 | 11.9 | -2.0 | -33.7 | 100 |
| 6 | 11.7 | -2.2 | -32.9 | 100 |
| 7 | 11.4 | -2.4 | -32.1 | 100 |
| 8 | 11.2 | -2.6 | -31.5 | 100 |
| 9 | 10.9 | -2.7 | -30.8 | 100 |

### EXAMPLE 3

A fermentation was performed as described in EP3927837A1 and as indicated in example 1 above. The resulting fermentation broth was subjected to pre-treatment, solid-liquid separation and concentration steps, known in the art, yielding a fermentation derived solution with a pH value 1.5 - 1.6 units below the enzymes isoelectric point.

CIELAB color values of the fermentation derived solution were measured using a Lico 690 Spectral colorimeter. Measured samples were sterile filtered using 0.2 µm syringe filters ensuring optically clear solutions. 80 g of the fermentation derived solution without sulfate was split into four parts of 20 g each. 0.25, 0.5, 1.0, and 1.5 g of the same strong base anionic exchange resin used in example 1 were added to the four solutions in stirred beakers, respectively, and incubated for approx. 24h. CIELAB color values of the solutions after incubation were measured as described above.

A further experiment was performed. Again, 80 g of the above-described fermentation derived solution without sulfate (< 0,05 g/kg) was spiked with approx. 5 g/kg SO4. Resulting solution was split into four parts of 20 g each. 0.25, 0.5, 1.0, and 1.5 g of the same strong base anionic resin were added to the four solutions in stirred beakers, respectively, and incubated for approx. 24h. CIELAB color values of the incubated solutions were measured as described above. Further, SO4 concentrations before and after incubation were measured spectroscopically.

Table 1 shows the reduction of color of the experiment without spiked SO4 in the fermentation derived solution as well as the reduction of color and SO4 of the experiment with 5 g/kg SO4 spiked into the fermentation derived solution.

Delta values indicate the increase (positive values) or decrease (negative values) in respective values and/or concentrations due to the incubation with respective amounts of the strong base anion exchange resin.

| Ferm. Derived solution | Amount of resin added | Delta L | Delta A | Delta B | Delta SO₄ |
|---|---|---|---|---|---|
| [g] | [g] | | | | [g/kg] |
| 20 | 0.25 | 7.2 | -3.1 | -20.3 | w/o spiked SO4 |
| 20 | 0.25 | 8.0 | -3.3 | -22.5 | -0.3 |
| | | | | | |
| 20 | 0.5 | 9.0 | -3.4 | -25.3 | w/o spiked SO4 |
| 20 | 0.5 | 9.1 | -3.4 | -25.9 | -0.7 |
| | | | | | |
| 20 | 1 | 12.2 | -3.1 | -35.3 | w/o spiked SO4 |
| 20 | 1 | 10.4 | -3.4 | -29.8 | -1.3 |
| | | | | | |
| 20 | 1.5 | 12.8 | -2.8 | -37.8 | w/o spiked SO4 |
| 20 | 1.5 | 10.9 | -3.3 | -32.5 | -2.0 |

The results clearly indicate that both colored compounds and sulfate adsorb to the strong base anion exchange resin. Surprisingly, the adsorption of sulfate did not influence the removal of colored compounds, which is indicated by almost identical changes of the L, A and B values in both experiments.

## Claims

1. A method for purifying a fermentation-derived solution of an enzyme of interest comprising at least the following steps:
a) providing a fermentation-derived solution of an enzyme of interest comprising 0.1 to 40 g/kg sulfate and 0 to 20 g/kg phosphate;
b) contacting the solution of the enzyme of interest with an anion exchange resin;
c) obtaining a purified enzyme solution, wherein the concentration of sulfate and/or phosphate is below the concentration of sulfate and/or phosphate in step a) and wherein the purified enzyme solution has a CIELAB L-value of above 60.

2. The method according to claim 1, wherein the purified enzyme solution comprises less than 0.05 g/kg phosphate.

3. The method according to any of claims 1 or 2, wherein the enzyme of interest is a protease.

4. The method according to any of claims 1 to 3, wherein the purified enzyme solution comprises less than 0.1 g/kg sulfate and /or less than 0.05 g/kg phosphate.

5. The method according to any one of claims 1 to 4, wherein the L-value of the purified enzyme solution in step c) is at least 5 units higher than the L-value of the fermentation derived solution of the enzyme of interest in step a), preferably at least 7, more preferably at least 10 units higher than the L-value of the fermentation derived solution of the enzyme of interest in step a).

6. The method according to any one of claims 1 to 5, wherein the enzyme of interest is in an inactivated state throughout the purification process.

7. The method according to any one of claims 1 to 6, wherein the method further comprises one or more of the following additional steps: ultracentrifugation, evaporation, ultrafiltration, centrifugation, spray draying, granulation, freeze-drying, stabilization, crystallization, precipitation, adsorption, chromatographic purification such as affinity chromatography, and the like.

8. The method according to any one of claims 1 to 7, wherein the anion exchange is performed on an anion exchange column comprising an anion exchange resin that comprises ammonium groups or substituted ammonium groups; preferably quaternary ammonium groups; more preferably, the anion exchange column comprises an anion exchange resin further comprising a polymer matrix, such as a styrenic or acrylic matrix.

9. The method according to claim 8, wherein the anion exchange in step b) is performed using a bed volume between 0.5 m³ and 20 m³.

10. The method according to any one of claims 3 to 9, wherein the purified enzyme solution comprises a protease in a concentration of 2 to 400 g/kg, preferably 30 to 150 g/kg.

11. The method according to any one of claims 3 to 10, wherein the protease in step c) has an activity of at least 95 % of the protease in the fermentation derived solution in step a).

12. The method according to any one of claims 1 to 11, wherein the pH of the fermentation derived solution of an enzyme of interest is at least 0.5 pH units below the enzyme's isoelectric point.

13. The method according to any one of claims 1 to 12, wherein the CIELAB a- and/or the b-value of the purified enzyme solution is lower than the CIELAB a- and/or the b-value of the fermentation-derived solution of an enzyme of interest.

14. The method according to claim 13, wherein the CIELAB a-value of the purified enzyme solution in step c) is at least 1 unit lower than the a-value of the fermentation derived solution of the enzyme of interest in step a), preferably at least 2 units, more preferably at least 3 units lower than the a-value of the fermentation derived solution of the enzyme of interest in step a); and/or
wherein the CIELAB b-value of the purified enzyme solution in step c) is at least 5 units lower than the b-value of the fermentation derived solution of the enzyme of interest in step a), preferably at least 10 units, more preferably at least 15 units lower than the b-value of the fermentation derived solution of the enzyme of interest in step a).

15. A solution of an enzyme of interest obtained by or obtainable by the method according to any of the preceding claims.
